(19) Europäisches Patentamt
European Patent Office
Office européen des brevets

(11) **EP 4 258 268 A1**

(12) **EUROPEAN PATENT APPLICATION**

(43) Date of publication:
**11.10.2023 Bulletin 2023/41**

(51) International Patent Classification (IPC):
**G16B 30/00** (2019.01)

(21) Application number: **22166649.8**

(52) Cooperative Patent Classification (CPC):
**G16B 30/00**

(22) Date of filing: **05.04.2022**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **BIOMÉRIEUX**
**69280 Marcy L'Etoile (FR)**

(72) Inventors:
• **MAHE, Pierre**
  **38250 Lans En Vercors (FR)**

• **EL AZAMI, Meriem**
  **69210 L'Arbresle (FR)**
• **DANCETTE, Magali**
  **69280 Marcy l'Etoile (FR)**
• **TOURNOUD, Maud**
  **38360 Sassenage (FR)**
• **GRIFFON, Aurélien**
  **69009 Lyon (FR)**

(74) Representative: **bioMérieux PI Groupement mandataires**
**bioMérieux**
**69280 Marcy l'Etoile (FR)**

(54) **DETECTION OF A GENOMIC SEQUENCE IN A MICROORGANISM GENOME BY WHOLE GENOME SEQUENCING**

(57) A method for detecting target genomic sequences at predetermined positions in a sequenced genome of a microbial organism in the form of reads. The method comprises computing a local sequencing depth of the genome in a neighborhood of the position of a target genomic sequence, said computing comprising the steps of a) detecting in the reads a set of digital genomic sequences from at least one reference genome of the microbial organism, b) generating of third set of digital genomic sequences comprising reads and digital genomic sequences of the second set belonging to the neighborhood c) counting the number of copies of each digital genomic sequences of the third set of digital genomic sequences, and d) computing the local sequencing depth as being equal to the maximum of the counted numbers of copies.

k-mer detection threshold = fraction of the *local* sequencing depth

**Fig. 1**

**Description**

**FIELD OF THE INVENTION**

The invention relates to the field of microbiological molecular analysis, in particular to the identification of genomic sequences in a genome that has been sequenced using whole genome sequencing technology.

Advantageously, the invention applies to the identification of a sub-level species taxon identification to which a microbe belongs, e.g. a clonal complex of SARS-COVID-2 virus.

**STATE OF THE ART**

Next generation (whole genome or targeted) sequencing ("NGS"), e.g. as carried out by Illumina and Oxford Nanopore Technologies platforms, heavily transforms microbiological analysis of patient, food or environmental samples by traversing usual microbiological analysis workflow drawbacks such as low agnosticism level regarding the microbes in the sample, workflow duration or workflow complexity to name of few. If NGS technology shows many advantages, it however requires complex bioinformatics pipelines to process reads generated by sequencing platforms. Indeed, generally speaking, pipelines relies on specific genomic sequence detection, for instance to figure out the clonal complex belonging of a virus or the antibiotic susceptibility of a bacterium, which may be a challenging task because the genomic sequence may not be present as such in the reads or because of a high sequencing error rate.

To affirm the presence of a specific sequence in a genome, a natural approach consists in first computing the number of copies of the sequence in the reads and the average sequencing depth over the genome, and then conclude the presence of the sequence if its number of copies exceeds a predetermined fraction of the average sequencing depth, as described in patent applications WO2021180771 and WO2021180768. The concept behind this kind of pipelines relies on the fact that if one finds a limited number of copies of a specific sequence comparatively to the overall number of sequences generated by the sequencing platforms, it means those copies come from sequencing errors. One can thus concludes the specific sequence is actually not present in the genome.

However, in addition to a sequencing depth that varies along the genome because of the WGS technics itself, an enrichment step aiming at multiplying the number of some genome portions may be carried out before the sequencing step. This is for example the case of SARS-CoV2 variant identification, where sequencing generally occurs after a preliminary step of genome amplification done by massively multiplex PCR. The sequencing depth may thus vary in large extent along the genome. In one hand, if the actual sequencing depth at the targeted sequence position in the genome is a lot lower than the average depth, the pipeline is likely to deliver a false negative. In the other hand, if the actual sequencing depth at the targeted sequence position in the genome is a lot higher than the average depth, the pipeline is likely to deliver a false positive.

**DISCLOSURE OF THE INVENTION**

The purpose of the present invention is to provide a robust method for detecting specific genomic sequences in a microbial genome (hereafter "target genomic sequences") in a set of reads generated by a NGS workflow even in case of highly variable sequencing depth.

To this end, an object of the invention relates to a method for detecting target genomic sequences at predetermined positions in a genome of a microbial organism, comprising:

- preparation of a sample comprising at least one copy of the genome of the microbial organism;
- sequencing of said sample in order to generate a first set of digital genomic sequences corresponding to the genome of the microbial organism;
- and for each of the target genomic sequence, computer implemented step of:

    A. computing a local sequencing depth of the genome of the microbial organism in a neighborhood of the position of said target genomic sequence;
    B. detecting that said target genomic sequence is present in the genome of the microbial organism if a number of copies of said target genomic sequence is higher that a predetermined fraction of the computed local sequencing depth,

wherein the step of computing the local sequencing depth comprises the steps of:

    A1. detecting, in the first set of digital genomic sequences, a second set of digital genomic sequences, said second set comprising digital genomic sequences from at least one reference genome of the microbial organism;

A2. generating of third set of digital genomic sequences comprising:

> i. digital genomic sequences of the first set belonging to the neighborhood of the position of said target genomic sequence;
> ii. digital genomic sequences of the second set belonging to the neighborhood of the position of said target genomic sequence;

A3. counting the number of copies of each digital genomic sequences of the third set of digital genomic sequences; and

A4. computing the local sequencing depth as being equal to the maximum of the counted numbers of copies.

In the meaning of the invention, a microbial organism refers to a virus, a bacterium, a yeast or a fungus. In the meaning of the invention, a genome relating to a bacterium, a yeast or a fungus comprises the chromosomic and plasmid part.

In other words, the detection according to the invention relies on the synergy of the following concepts:

> i. The presence of a target genomic sequence in the microbial genome is confirmed if its number of copies in the reads exceeds a fraction of a local sequencing depth. By doing so, the variation of the actual sequencing depth along the genome is taken into account thereby reducing the rate of false positives and negatives one obverses when using an average sequencing depth;
> ii. The estimation of the local sequencing depth relies on counts of the target genomic sequence in the reads, but also on counts of other target genomic sequences in its neighborhood, as well as counts of similar genomic sequences possibly coming from mutations of the target genomic sequence observed in genomes of the microbial organism. The estimation of the local sequencing depth is finally defined as the highest number of counts found in the neighborhood of the target sequence, which more robustly reflects the actual composition of the genome of the organism at the position of the target sequence than the average sequencing depth along the entire genome.
> iii. In addition, the counts may not perfectly reflect the actual local sequencing depth due to sequencing errors. As a result, one cannot merely state that the target sequence is not present in the genome if it is not the most abundant sequence in its neigborhood. By using a fraction of the estimated local sequencing depth, and thus a fraction of the most abundant sequence in the neighborhood of the target sequence, the sequencing error is explicitly taken into account. One notes here the synergistic relationship between local sequencing depth and error rate consideration since the latter cannot be managed without first defining a local sequencing depth.

According to one embodiment of the invention, the target genomic sequences are k-mers which are constituent of variables of a model for predicting the belonging of the microbial organism to a clonal complex. In particular, the microbial organism is a coronas virus and the length of the k-mers is comprised between 15 and 25.

According to one embodiment of the invention, the genomic sequences of the second set of genomic sequences are regularly spaced.

The invention also relates to a system for detecting target genomic sequences at predetermined positions in a genome of a microbial organism, comprising a computing unit configured to carry out the above described steps A and B. The invention also relates to a computer readable medium storing instructions for executing a method performed by a computer, the method comprising the above described step A and B.

## BRIEF DESCRIPTION OF THE FIGURES

The invention will be better understood upon reading the description which follows, given solely by way of example, and made in relation to the appended drawings, in which identical reference symbols designate identical or similar elements, and in which:

- **Figure 1** illustrates the adaptive threshold definition for a given k-mer (shown circled by a bold contour) based on the number of occurrences of its neighboring k-mers (shown circled by a dashed contour);
- **Figure 2** shows the performance and the support of model chosen by cross-validation, for k = 21;
- **Figure 3** illustrates that the k-mers selected in the model naturally capture lineage-defining mutations;
- **Figure 4** gives the confusion matrix obtained on the assembly test set, involving 2000 genomes per clade;
- **Figure 5** gives the confusion matrices obtained on the ONT test dataset when predicting from all the reads available (endpoint strategy), relying or not on the adaptive thresholding method;
- **Figure 6** illustrates the impact of adaptive thresholding according to the invention on a sample that was misclassified using the average thresholding strategy;

- **Figure 7** gives these minimal sequencing depth and the difference in the reached performance for each clade

## DETAILED DESCRIPTION OF THE INVENTION

A. Embodiment of the invention carried out for detecting clonal complex of SARS-CoV-2 virus

An embodiment of the invention is now described in relation with the prediction of a clonal complex to which belongs a SARS-COV-2 virus infecting a patient using reads generated by ONT platforms.

A.1 Introduction

Since the start of the COVID-19 pandemic at the end of 2019, many variants of the original SARS-CoV-2 strains emerge regularly (van Dorp et al., 2020). Some of these variants exhibit highly modified properties of the virus such as a higher transmissibility and associated disease severity, or a reduced effectiveness of treatments or vaccines, or a reduced performance of diagnostic tools, or other public health and social measures. The monitoring and the assessment of these various SARS-CoV-2 variants has therefore been key in the management of the ongoing COVID-19 pandemic (WHO, 2021). In order to prioritize global monitoring and research, the world health organization (WHO) and the Centers for Disease Control and Prevention (CDC) identify specific Variants of Interest (VOIs) and Variants of Concern (VOCs) that pose an increased risk to global public health and for which additional measures have to be implemented to prevent their spread (WHO, 2021; CDC, 2021; Parums, 2021). Sequencing has played a fundamental role in identifying the SARS-CoV-2 as the causative agent of COVID-19 (Wu et al., 2020) and has now become essential to understanding the virus dynamics and the monitoring of its spread. Two main reference tools, Pangolin (O'Toole et al., 2021) and Nextclade (Aksamentov et al., 2021), are used to identify SARS-CoV-2 variants from assembled genomes. Both tools focus on the detection of point-mutations within various proteins (including the spike protein).

Next generation sequencing platforms, usually used for routine whole genome sequencing, have been successfully used for SARSCoV- 2 sequencing. These include the mainstream Illumina platforms and Oxford Nanopore Technologies (ONT). Although both technologies allow multiple samples to be sequenced together (multiplexing), Illumina sequencing remains too long and costly for a timely diagnostic. ONT compact instruments offer a faster turnaround by analyzing the sequencing reads in real time (Magi et al., 2017). This real-time feature can be critical in many clinical applications, for instance for therapy adjustment where, due to the presence of the SARS-CoV-2 virus, the targeted virus within the same host can mute and escape therapy. However ONT reads exhibit high error rates (e.g. $\approx 5\%$ with R9.4 flowcells) and therefore the use of appropriate bio-informatics pipelines is necessary to obtain accurate results, especially for the detection of point-mutations (Delahaye and Nicolas, 2021). Finally, for SARS-CoV-2, prior to sequencing library preparation, polymerase chain reactions (PCRs) that generate amplicons have been used to amplify SARS-CoV-2 genetic material. The most widely used scheme for SARS-CoV-2 sequencing is currently the tiling amplicon-based approach designed by the ARTIC Network (Quick et al., 2017). In this approach primer sets that target amplicons of different lengths, typically 400 - 2000 bases have been designed to optimally cover the SARS-CoV-2 genome. Several primer sets are now commercialized and used for SARS-CoV-2 sequencing (Itokawa et al., 2020;Tyson et al., 2020). However, depending on the used primer kit, differences in primer efficiency and amplicon yield are observed. This in turn leads to a non-uniform sequencing depth along the genome (Charre et al., 2020; Liu et al., 2021).

The inventors propose a robust approach based on ONT sequencing, requiring a limited amount of sequencing data, thereby allowing ultra-fast variant identification with high accuracy. This approach relies on a k-mer representation of SARS-CoV-2 genomes and sparsity promoting machine learning models to identify concise genomic signatures that can efficiently and accurately be detected from reads. To enhance the accuracy of the models the inventor approach specifically deal with the high error rates of ONT sequencing and the non-uniform sequencing depth obtained with the ARTIC protocol by relying on a local estimate of the sequencing depth to optimally detect the k-mers embedded in the signatures.

A.2. Dataset

Two datasets are gathered for this study. The first one involves assembled SARS-CoV-2 genomes from the GISAID database (Khare et al., 2021). It is used to learn and validate a machine learning model. The second dataset consists of ONT sequencing reads and matching assembled genomes. It is used to illustrate the feasibility of predicting directly from reads in realtime and without any loss in performance when compared with a prediction from assembled genomes.

A.2.1. Assembled genomes

At the heart of the approach is a machine learning model trained to identify SARS-CoV-2 variants. In this work, it is considered the Nextstrain nomenclature to define variants and rely on assembled genomes taken from the GISAID database to learn and validate the model. More precisely, the approach relies on an export of the database made on May 14th 2021 consisting of 1:508:656 genomes and representing 13 clades. From this database, it is picked 2000 genomes for training and 2000 genomes for independent validation.

A.2.2. ONT reads

In order to demonstrate the feasibility of ultra-fast identification of SARSCoV- 2 variants from ONT reads, it is gathered a smaller dataset with 100 genomes per clade. ONT reads were downloaded from the Sequence Read Archive (SRA) and the corresponding assemblies were extracted from the GISAID database for comparison purposes. None of the genomes included in this dataset were part of the dataset used to train and validate the machine learning model. Table 1 provides the number of assembled genomes per clade used to train and validate the model and the number of ONT genomes (reads) used to test the model. The first column gives the clade names, the second one corresponds to the total number of genomes available in the GISAID export, column 2 and 3 correspond to the number of genomes used to train and validate the model and column 4 corresponds to ONT reads dataset used to test the model.

A.3. Method

A.3.1. genomic sequence representation

The inventor approach relies on a k-mer representation of the genomic sequences of the SARS-CoV-2. The DBGWAS software is used to compute the k-mer representation, as described in Jaillard et al. (2020), patent applications WO2021180771 and WO2021180768. Briefly, for a given k value, a De Bruijn Graph is built from all the assemblies within the training dataset. The nodes of this graph correspond to k-mers, which are compacted into so-called unitigs for perfectly conserved regions, leading to variable length k-mers. To further reduce the number of dimensions, equivalent unitigs are grouped together into features. The final representation for given genome is obtained by the binary vector encoding the presence or absence of all these features, and is used as input to the machine learning model.

Table 1

| clade | total | train | validation | Test-ONT |
|---|---|---|---|---|
| 19A | 19412 | 2000 | 2000 | 97 |
| 19B | 11350 | 2000 | 2000 | 97 |
| 20A | 183169 | 2000 | 2000 | 100 |
| 20A(EU2) | 28748 | 2000 | 2000 | 97 |
| 20B | 192425 | 2000 | 2000 | 98 |
| 20C | 150519 | 2000 | 2000 | 91 |
| 20D | 9681 | 2000 | 2000 | 100 |
| 20E(EU1) | 160869 | 2000 | 2000 | 100 |
| 20F | 12841 | 2000 | 2000 | 100 |
| 20G | 88265 | 2000 | 2000 | 17 |
| 20H/501Y.V2 | 16348 | 2000 | 2000 | 99 |
| 20I/501Y.V1 | 618463 | 2000 | 2000 | 100 |
| 20J/501Y.V3 | 16227 | 2000 | 2000 | 14 |
| Total | 1508317 | 26000 | 26000 | 1010 |

A.3.2. Model training from assemblies

A lasso-penalized multinomial logistic regression model is trained, said model aiming to identify the variant of a genome, as defined by the Nextclade nomenclature, from the above k-mer based representation.

Multinomial logistic regression is a widely used generalized linear model addressing multiclass classification problems. In our case, it consists of building a set of K linear functions $f_k(.)$ defined, for a genome represented by a vector x ∈ $\{0,1\}^p$, as:

$$f_k(x) = \beta_0^{(k)} + \sum_{j=1}^{p} \beta_j^{(k)} x_j$$

where $K$ is the number of Nextstrain clades considered (13 in this study), $p$ is the number of features involved in the k-mer representation and $\beta_0^{(k)}$ and $\beta_j^{(k)}$ are the model coefficients. The model defines the probability of belonging to each one of the K clades as:

$$P(Y = k|x) = \frac{exp(f_k(x))}{\sum_{l=1}^{K} exp(f_l(x))} \qquad \text{Eq 1}$$

for $k \in \{1, ..., K\}$, and the final prediction corresponds to the clade with the highest probability.

To estimate the model coefficients and simultaneously select a limited number of features involved, a standard Lasso-penalized maximum-likelihood procedure is used. More formally, starting from a panel *of n* genomes $(x_i, y_i)$, where $y_i \in \{1, ..., K\}$ denotes the actual clade of the ith genome and $x_i \in \{0,1\}^p$ its k-mer representation, the following optimization problem is solved:

$$B^* = arg \min_{B \in \mathbb{R}^{(p+1) \times K}} \sum_{i=1}^{n} - \log P(Y = y_i|x_i) + \lambda \sum_{k=1}^{K} \sum_{j=1}^{p} |B[j,k]|$$

where $B[j, k] = \beta_j^{(k)}$ and - log $P(Y = y_i|x_i)$ defined according to Eq. 1 as:

$$\log P(Y = y_i|x_i) = f_{y_i}(x_i) - \log\left(\sum_{l=1}^{K} exp(f_l(x_i))\right)$$

The regularization parameter $\lambda$ controls the trade-off between prediction accuracy and the sparsity of the model, and is chosen by cross-validation to maximize model accuracy. In practice, we relied on the R software, and more precisely on the glmnet package (Friedman et al., 2010), to estimate the model coefficients and optimize the regularization parameter.

A.3.3. Prediction from assemblies

The prediction step simply amounts to detecting the presence of the features selected by the model (a set of unitigs) in the sequencing data. To do so from an assembled genome, in three steps process is carried out. As unitigs can have a variable length (between 21 and 41 in this study), it is first detected all k-mers of length 21 using MUMmer (Marçais et al., 2018) and then called a unitig present when all its constitutive k-mers are detected without errors (no mismatches). Finally, when several unitigs define a given feature, it is required that all the unitigs be present.

A.3.4. Prediction from reads

The strategy used for assembly-based prediction was adapted to predict directly from reads by including a threshold on the number of occurrences of each individual k-mer, while keeping unchanged the rest of the procedure.

As describe above different approaches can be used to define the k- mer detection threshold. A first natural strategy is to rely on a unique threshold T defined as a fraction $\alpha$ of the average sequencing depth. This average sequencing depth is estimated by dividing the total number of sequenced base-pairs ($N_{bps}$) by the length of the reference SARS-CoV-2 genome MN908947 (equals to 29903 bps, see e.g. Wu et al. (2020)) :

$$T = \alpha \times \frac{N_{bps}}{29903}$$

However, as described in the introduction, the ARTIC protocol used for SARS-CoV-2 sequencing includes an enrichment step which results in a sequencing depth that varies along the genome. In addition the sample composition can also lead to non uniform sequencing depth especially in case of contamination.

To address this limitation the invention proposes an alternative strategy relying on an adaptive threshold. This approach relies on a local estimation of the sequencing depth, to define k-mer specific detection thresholds. For the local estimation of the sequencing depth, an embodiment of the invention advantageously leverages the k-mers used by the fastv software (Chen et al., 2020) that is designed to assess the quality of the sequencing data using generated k-mers regularly spaced along a given genome. These k-mers generated by fastv are thus regularly spaced along the SARS-CoV-2 genome and therefore offer a natural proxy to estimate the local sequencing depth. More precisely, the embodiment of the invention proceed as follows:

1. first map both the fastv k-mers and the k-mers involved in the model on the reference SARS-CoV- 2 genome MN908947 (Wu et al., 2020), and store for each k-mer $k_i$ its position $pos(k_i)$ on the reference genome.

2. for a given window size $\delta$, and for each k-mer $k_i$, a neighborhood $\mathcal{N}_i$ is defined according to Eq. 2 integrating both the k-mers of the model and the fastv k-mers that are within a distance $\delta$ from the k-mer $k_i$ :

$$\mathcal{N}_i = \left\{ k_j \in \mathcal{K}, j \neq i : \left| pos(k_j) - pos(k_i) \right| < \delta \right\} \qquad \text{Eq. 2}$$

where K denotes the union of the set of fastv k-mers and the set of k-mers involved in the prediction model.

3. the local sequencing depth is estimated as being the maximum number of k-mer occurrences *(occ)* observed within the neighborhood $\mathcal{N}_i$ , as both fastv k-mers and the k-mers from the model may capture different mutations with respect to the reference genome.

4. finally, as expressed in Eq. 3, a threshold $T_i$ associated to the k-mer $k_i$ is defined as a fraction $\alpha$ of this local estimate of the sequencing depth. Of note, however, depending on the size of the neighborhood $\delta$, a k-mer may remain isolated. In this case, we rely on the median value of all the k-mers occurrences (both signatures and fastv) to estimate the local sequencing depth.

$$T_i = \begin{cases} \alpha \times max\{occ(k_j)\}, & \forall k_j \in \mathcal{N}_i \text{ if } \mathcal{N}_i \neq \emptyset \\ median\{occ(k_j)\}, & \forall k_j \in \mathcal{K} \text{ otherwise} \end{cases} \qquad \text{Eq. 3}$$

In practice, $\alpha$ and $\delta$ are two hyper-parameters that are advantageously optimized to enhance performance.

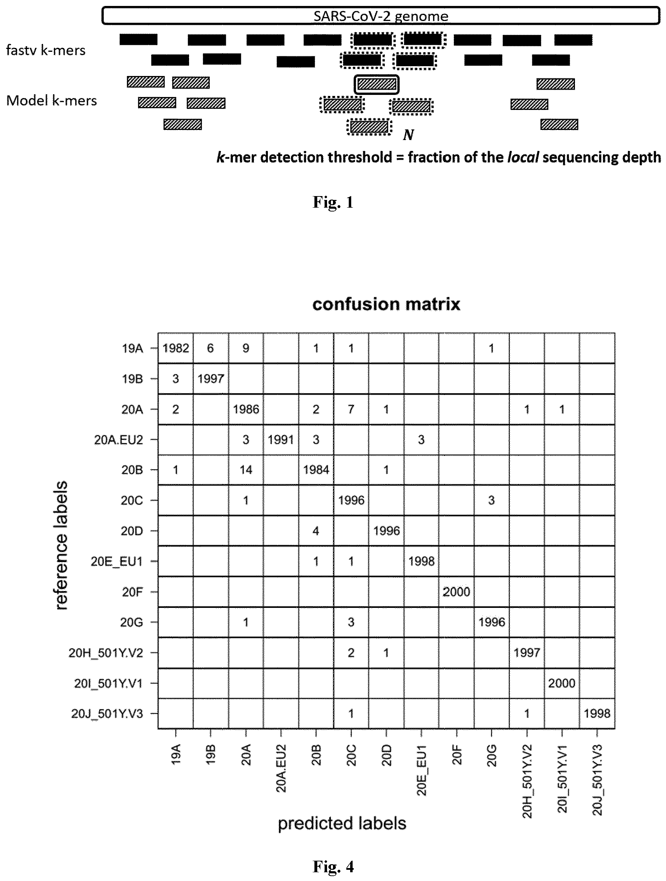

Figure 1 illustrates the different steps used to define such an adaptive k-mer detection threshold based on k-mers derived from a reference genome and the model k-mers.

A.3.5. Real-time / streaming analysis

Two prediction settings are advantageously considered when identifying the variants from ONT reads. The end-point setting corresponds to making a single prediction from all the sequencing reads available at the end of the sequencing experiment, as would be done in a traditional Illumina-like sequencing workflow.

However, as the ONT workflow offers the possibility of processing the sequencing data in real-time, we evaluated the added value of this unique feature of ONT sequencing by simulating the real-life situation where the reads are produced in real-time by the sequencer. For this purpose, we split the sequencing data downloaded from the SRA in chunks of 1000 reads. This allowed the study of the evolution of the prediction performance as the sequencing depth and sequencing time increase.

A.4. Results

A.4.1. Cross-validation performance

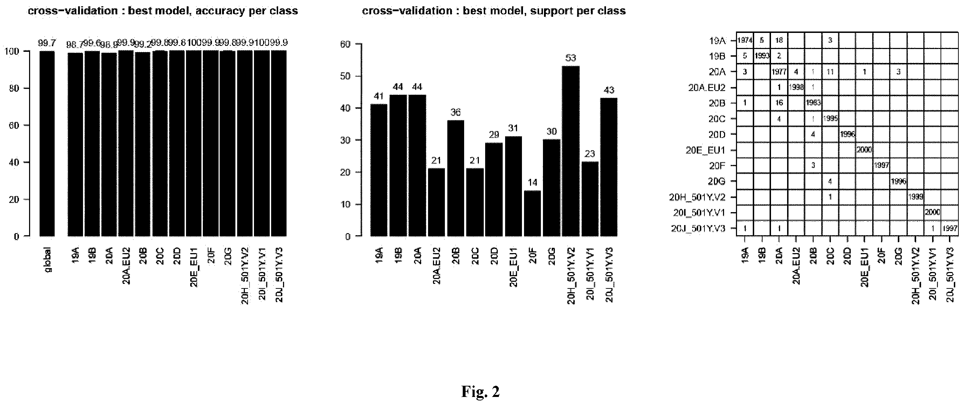

The prediction model is trained using several values of k between 11 and 31. The best k value is chosen based on cross-validation results. The inventors observe little influence of k value on the performance and the support of the model. The value of 21 is chosen for its best trade-off in terms of performance and sparsity of the model. The total number of features that are involved in the model was 430. Figure 2 shows the performance and the support of model chosen by cross-validation, for k = 21.

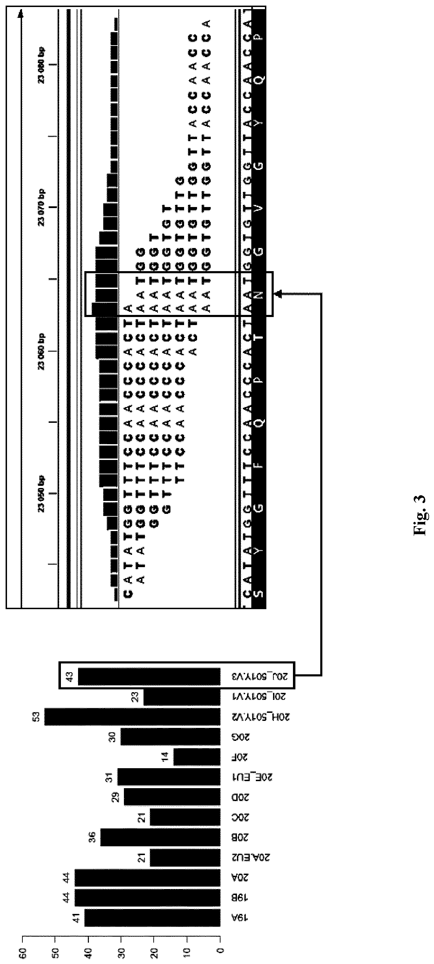

Figure 3 illustrates that the k-mers selected in the model naturally capture lineage-defining mutations. In this example, k-mers corresponding to the 20J/501Y.V3 clade are mapped against the reference genome of SARS-CoV-2. This set of k-mers capture the AAT in position 23063, which correspond to the absence of the well-known N501Y mutation in the Spike protein and whose presence is associated with the 20J/501Y.V3 clade.

A.4.2. Performance on assemblies

Figure 4 gives the confusion matrix obtained on the assembly test set, involving 2000 genomes per clade. The overall accuracy of the model is 99,7%. This performance level is very consistent with cross-validation performance showing the good generalization properties of the machine learning model and the process of detecting k-mer based on local sequencing depth according to the invention.

A.4.3. Performance on ONT reads

The ONT reads from the test dataset were processed using our prediction pipeline by considering both the end-point and the streaming prediction strategies. For comparison purposes, the prediction model is also tested on the corresponding assemblies from GISAID. For the adaptive thresholding method, the neighborhood size and threshold are set arbitrarily to $\delta = 31$ and $\alpha = 0:5$, respectively. These two hyper-parameters are fine-tuned to obtain appropriate level of performance by relying on a cross-validation procedure.

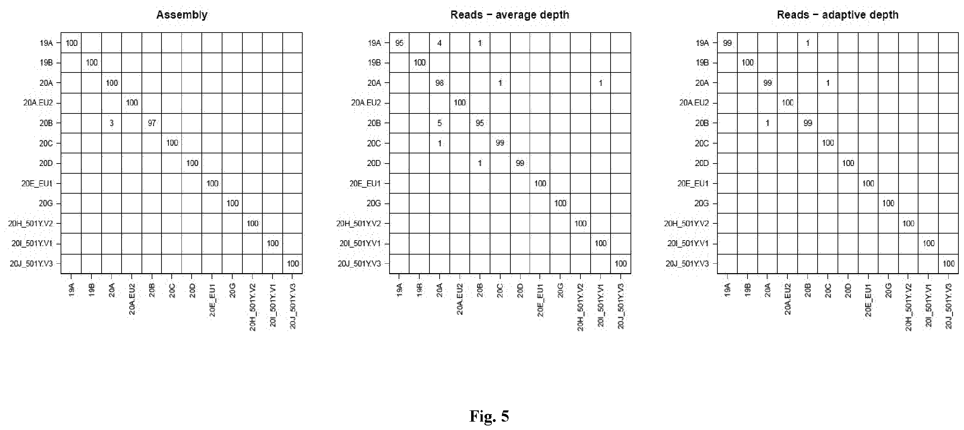

End-point performance: Figure 5 gives the confusion matrices obtained on the ONT test dataset when predicting from all the reads available (endpoint strategy), relying or not on the adaptive thresholding method. These results are compared to the confusion matrix obtained from assembled genomes (shown on the left-hand side).

The overall prediction accuracy for the assembled genomes is 99:7% which indicates that this small-scale dataset is representative of the validation dataset which is a performance level equivalent to that of assembly-based prediction.

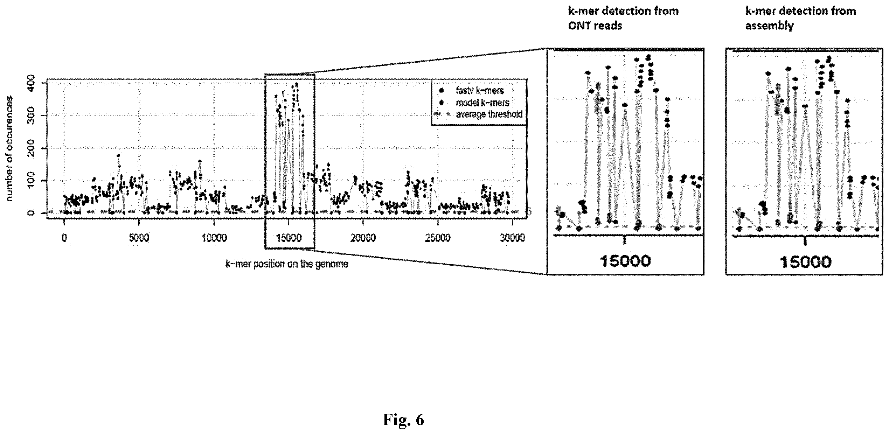

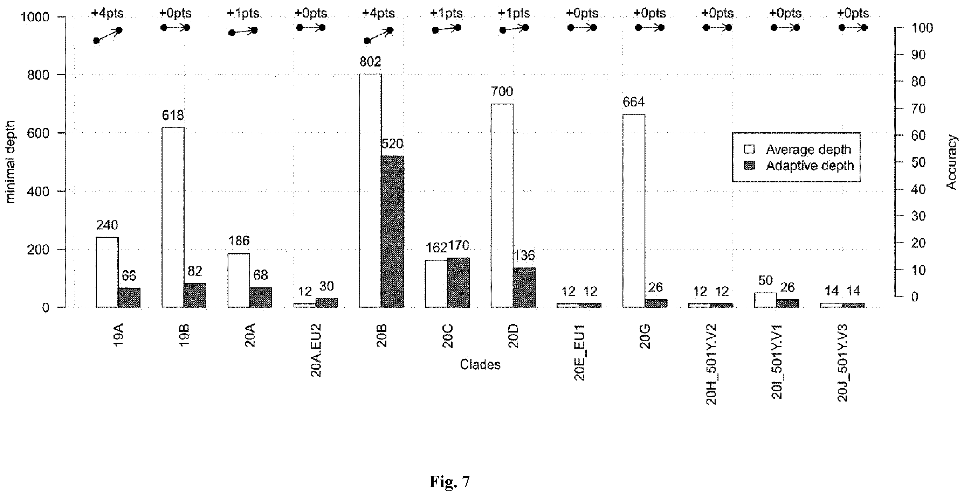

Figure 6 illustrates the impact of adaptive thresholding on a sample that was misclassified using the average thresholding strategy, but correctly classified after adaptive thresholding. It highlights how the adaptive thresholding strategy impacted k-mer detection. In all three panels, the black points correspond to fastv k-mers, whereas the blue points correspond to the k-mers selected by the model. The dashed orange line corresponds to the average k-mer detection threshold. In both zoomed-in panels, model k-mers that were detected for this sample are highlighted in yellow. The red points correspond to model k-mers that were only detected using the average threshold strategy. In particular, several k-mers occurring in the high sequencing depth region (shown in red, around position 15000) that were called present with the global threshold ended up not being called anymore using adaptive thresholding, in agreement with the calls made from the corresponding assembled genome. The analysis of the streaming performance shows that for both depth estimation approaches, the prediction performance reaches the plateau defined by the end-point accuracy before the end of the sequencing experiment. For the average thresholding method, the minimal depth to reach the plateau is around 750x, whereas for the adaptive thresholding strategy the minimal depth is around 150x. The inventors observe that in the streaming mode, the performance obtained with the adaptive depth method is also more stable than that of the average depth method. To better illustrate both the difference in performance and speed, we computed the minimal sequencing depth required to reach the end-point performance for each clade. Figure 7 gives these minimal sequencing depth and the difference in the reached performance for each clade. This result clearly shows that not only higher performance levels are reached when relying on the adaptive depth method but also lower sequencing depths are needed to reach the end-point performance.

As one can note from the above a method that relies on k-mers and sparsity promoting machine learning models to identify SARS-CoV-2 variants directly from ONT reads. By using a local sequencing depth estimation, one observe a great reduction of the amount of sequencing data required to confidently detect k-mers, while maintaining a level of variant identification performance comparable to that obtained from assembled genomes. In particular, it is demonstrated that this performance can be reached for an average sequencing depth as low as 150X, which could be reached in less than one minute of sequencing time if samples were not multiplexed on an ONT flowcell. Strikingly, not taking into account such a local, adaptive, strategy leads to lesser performance and a 5 fold increase in the required amount of sequencing data.

Variant identification can then be obtained in a few minutes of sequencing time, depending on the amount of sample multiplexing. This therefore paves the way to timely diagnostics, which can improve patient care in the context of the COVID-19 pandemic.

A.5. The processing done on reads to figure out the clonal complex of a Sars-CoV2 sample is a computer implemented method, for instance carried out by a computing unit comprising one or more (micro)processors, computer memory storage and RAM, and configured to store computer readable instructions that carry out the above described computation when run by the computing unit. The computing unit is for instance a personal computer, a server or computing cluster of a cloud-based computer system. Results of the processing are advantageously output on a display and/or stored in a laboratory information system.

B. Other embodiments

- it has been described the detection of k-mer in a genome. As it is readily understandable, the expression "k-mer" stands for a particular way of describing a genome, a portion thereof, or more generally a genomic sequence. As such, a k-mer is a genomic sequence so that its use in the description with regard to the prediction described above is not limiting the scope of the invention;
- it has been described an adaptive thresholding based on local sequencing depth computation with regard to a virus. Obviously, said method applies whatever the nature of the genome that may be genome from a bacterium, a yeast or a fungus. In particular, patent application WO2021180768 describes the prediction of the susceptibility to an antibiotic of a bacteria based on specific k-mers detection. The detection described in said application is advantageously carried out based on the invention;

- it has been described some specific values for the hyperparameters $\alpha$ and $\delta$. Advantageously, as described above, said hyperparameters are part of the variables of cross-validation technics, and their values chosen to optimize the performances of said cross-validation;
- it has been described the use of one reference genome. Advantageously multiple reference genomes, and preferably as much as possible reference genomes, are used to generated the reference k-mers.

**REFERENCES**

Aksamentov, I. et al. (2021). Nextclade: clade assignment, mutation calling and quality control for viral genomes. Journal of Open Source Software, 6(67), 3773.

CDC (2021). Sars-cov-2 variant classifications and definitions, extracted from: https: //www.cdc.gov/coronavirus/2019-ncov/variants/variantclassifications.html.

Charre, C. et al. (2020). Evaluation of ngs-based approaches for sars-cov-2 whole genome characterisation. Virus evolution, 6(2), veaa075.

Chen, S. et al. (2020). A computational toolset for rapid identification of sars-cov-2, other viruses, and microorganisms from sequencing data. bioRxiv.

Delahaye, C. and Nicolas, J. (2021). Sequencing DNA with nanopores: Troubles and biases. PLoS ONE, 16(10 October).

Friedman, J. et al. (2010). Regularization paths for generalized linear models via coordinate descent. Journal of Statistical Software, 33(1), 1-22.

Itokawa, K. et al. (2020). Disentangling primer interactions improves sars-cov-2 genome sequencing by multiplex tiling pcr. PloS one, 15(9), e0239403.

Jaillard, M. et al. (2020). Interpreting k-mer-based signatures for antibiotic resistance prediction. GigaScience, 9(10). giaa110.

Jouet, A. et al. (2021). Deep amplicon sequencing for culture-free prediction of susceptibility or resistance to 13 anti-tuberculous drugs. European Respiratory Journal, 57(3).

Khare, S. et al. (2021). Gisaid's role in pandemic response. China CDC Weekly, 3(49), 1049-1051.

Liu, T. et al. (2021). A benchmarking study of sars-cov-2 whole-genome sequencing protocols using covid-19 patient samples. Iscience, 24(8), 102892.

Magi, A. et al. (2017). Nanopore sequencing data analysis: state of the art, applications and challenges. Briefings in Bioinformatics, (June).

Marçais, G. et al. (2018). Mummer4: A fast and versatile genome alignment system. PLOS Computational Biology, 14(1), 1-14.

Meehan, C. J. et al. (2019). Whole genome sequencing of mycobacterium tuberculosis: current standards and open issues. Nature Reviews Microbiology, 17(9), 533-545.

O'Toole, et al. (2021). Assignment of epidemiological lineages in an emerging pandemic using the pangolin tool. Virus Evolution, 7(2). veab064.

Parums, D. V. (2021). Revised world health organization (who) terminology for variants of concern and variants of interest of sarscov- 2. Medical Science Monitor: International Medical Journal of Experimental and Clinical Research, 27, e933622-1.

Quick, J. et al. (2017). Multiplex pcr method for minion and illumine sequencing of zika and other virus genomes directly from clinical samples. Nature protocols, 12(6), 1261-1276.

Tyson, J. R. et al. (2020). Improvements to the artic multiplex pcr method for sars-cov-2 genome sequencing using nanopore. BioRxiv.

van Dorp, L. et al. (2020). Emergence of genomic diversity and recurrent mutations in sars-cov-2. Infection, Genetics and Evolution, 83, 104351.

WHO (2021). Tracking sars-cov-2 variants, extracted from: https://www.who.int/en/activities/tracking-sars-cov-2-variants/.

Wu, F. et al. (2020). A new coronavirus associated with human respiratory disease in china. Nature, 579(7798), 265-269.

## Claims

1. Method for detecting target genomic sequences at predetermined positions in a genome of a microbial organism, comprising:

   - preparation of a sample comprising at least one copy of the genome of the microbial organism;
   - sequencing of said sample in order to generate a first set of digital genomic sequences corresponding to the genome of the microbial organism;
   - and for each of the target genomic sequence, computer implemented step of:

      A. computing a local sequencing depth of the genome of the microbial organism in a neighborhood of the position of said target genomic sequence;
      B. detecting that said target genomic sequence is present in the genome of the microbial organism if a number of copies of said target genomic sequence is higher that a predetermined fraction of the computed local sequencing depth,

   wherein the step of computing the local sequencing depth comprises the steps of:

      A1. detecting, in the first set of digital genomic sequences, a second set of digital genomic sequences, said second set comprising digital genomic sequences from at least one reference genome of the microbial organism;
      A2. generating of third set of digital genomic sequences comprising:

         iii. digital genomic sequences of the first set belonging to the neighborhood of the position of said target genomic sequence;
         iv. digital genomic sequences of the second set belonging to the neighborhood of the position of said target genomic sequence;

      A3. counting the number of copies of each digital genomic sequences of the third set of digital genomic sequences; and
      A4. computing the local sequencing depth as being equal to the maximum of the counted numbers of copies.

2. Method according to claim 1, wherein the target genomic sequences are k-mers which are constituent of variables of a model for predicting the belonging of the microbial organism to a clonal complex.

3. Method according to claim 1, wherein the microbial organism is a coronas virus and the length of the k-mers is comprised between 15 and 25.

4. Method according to any one of the preceding claims, wherein the genomic sequences of the second set of genomic sequences are regularly spaced.

5. System for detecting target genomic sequences at predetermined positions in a genome of a microbial organism, comprising a computing unit configured to carry out steps A and B according to any one of the preceding claims.

6. A computer readable medium storing instructions for executing a method performed by a computer, the method comprising step A and B according to any one of claims 1-4.

k-mer detection threshold = fraction of the *local* sequencing depth

**Fig. 1**

## confusion matrix

| | 19A | 19B | 20A | 20A.EU2 | 20B | 20C | 20D | 20E_EU1 | 20F | 20G | 20H_501Y.V2 | 20I_501Y.V1 | 20J_501Y.V3 |
|---|---|---|---|---|---|---|---|---|---|---|---|---|---|
| 19A | 1982 | 6 | 9 | | 1 | 1 | | | | 1 | | | |
| 19B | 3 | 1997 | | | | | | | | | | | |
| 20A | 2 | | 1986 | | 2 | 7 | 1 | | | | 1 | 1 | |
| 20A.EU2 | | | 3 | 1991 | 3 | | | 3 | | | | | |
| 20B | 1 | | 14 | | 1984 | | 1 | | | | | | |
| 20C | | | 1 | | | 1996 | | | | 3 | | | |
| 20D | | | | | 4 | | 1996 | | | | | | |
| 20E_EU1 | | | | | 1 | 1 | | 1998 | | | | | |
| 20F | | | | | | | | | 2000 | | | | |
| 20G | | | 1 | | | 3 | | | | 1996 | | | |
| 20H_501Y.V2 | | | | | | 2 | 1 | | | | 1997 | | |
| 20I_501Y.V1 | | | | | | | | | | | | 2000 | |
| 20J_501Y.V3 | | | | | | 1 | | | | | 1 | | 1998 |

reference labels

predicted labels

**Fig. 4**

Fig. 2

Fig. 3

**Assembly**

| | 19A | 19B | 20A | 20A.EU2 | 20B | 20C | 20D | 20E_EU1 | 20G | 20H_501Y.V2 | 20I_501Y.V1 | 20J_501Y.V3 |
|---|---|---|---|---|---|---|---|---|---|---|---|---|
| 19A | 100 | | | | | | | | | | | |
| 19B | | 100 | | | | | | | | | | |
| 20A | | | 100 | | | | | | | | | |
| 20A.EU2 | | | | 100 | | | | | | | | |
| 20B | | | 3 | | 97 | | | | | | | |
| 20C | | | | | | 100 | | | | | | |
| 20D | | | | | | | 100 | | | | | |
| 20E_EU1 | | | | | | | | 100 | | | | |
| 20G | | | | | | | | | 100 | | | |
| 20H_501Y.V2 | | | | | | | | | | 100 | | |
| 20I_501Y.V1 | | | | | | | | | | | 100 | |
| 20J_501Y.V3 | | | | | | | | | | | | 100 |

**Reads – average depth**

| | 19A | 19B | 20A | 20A.EU2 | 20B | 20C | 20D | 20E_EU1 | 20G | 20H_501Y.V2 | 20I_501Y.V1 | 20J_501Y.V3 |
|---|---|---|---|---|---|---|---|---|---|---|---|---|
| 19A | 95 | | 4 | | 1 | | | | | | | |
| 19B | | 100 | | | | | | | | | | |
| 20A | | | 98 | | 1 | | | | | | 1 | |
| 20A.EU2 | | | | 100 | | | | | | | | |
| 20B | | | 5 | | 95 | | | | | | | |
| 20C | | | 1 | | | 99 | | | | | | |
| 20D | | | | | 1 | | 99 | | | | | |
| 20E_EU1 | | | | | | | | 100 | | | | |
| 20G | | | | | | | | | 100 | | | |
| 20H_501Y.V2 | | | | | | | | | | 100 | | |
| 20I_501Y.V1 | | | | | | | | | | | 100 | |
| 20J_501Y.V3 | | | | | | | | | | | | 100 |

**Reads – adaptive depth**

| | 19A | 19B | 20A | 20A.EU2 | 20B | 20C | 20D | 20E_EU1 | 20G | 20H_501Y.V2 | 20I_501Y.V1 | 20J_501Y.V3 |
|---|---|---|---|---|---|---|---|---|---|---|---|---|
| 19A | 99 | | | | 1 | | | | | | | |
| 19B | | 100 | | | | | | | | | | |
| 20A | | | 99 | | 1 | | | | | | | |
| 20A.EU2 | | | | 100 | | | | | | | | |
| 20B | | | 1 | | 99 | | | | | | | |
| 20C | | | | | | 100 | | | | | | |
| 20D | | | | | | | 100 | | | | | |
| 20E_EU1 | | | | | | | | 100 | | | | |
| 20G | | | | | | | | | 100 | | | |
| 20H_501Y.V2 | | | | | | | | | | 100 | | |
| 20I_501Y.V1 | | | | | | | | | | | 100 | |
| 20J_501Y.V3 | | | | | | | | | | | | 100 |

**Fig. 5**

**Fig. 6**

Fig. 7

Europäisches
Patentamt

European
Patent Office

Office européen
des brevets

# EUROPEAN SEARCH REPORT

Application Number

EP 22 16 6649

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | EP 3 879 537 A1 (BIOMERIEUX SA [FR]) 15 September 2021 (2021-09-15) * abstract * * paragraphs [0015], [0016], [0039], [0042], [0092], [0100] – [0102] * | 1-6 | INV. G16B30/00 |
| A,D | Chen Shifu ET AL: "A Computational Toolset for Rapid Identification of SARS-CoV-2, other Viruses, and Microorganisms from Sequencing Data", bioRxiv, 16 May 2020 (2020-05-16), XP055958278, DOI: 10.1101/2020.05.12.092163 Retrieved from the Internet: URL:https://www.biorxiv.org/content/10.1101/2020.05.12.092163v2.full.pdf [retrieved on 2022-09-06] * the whole document * | 1-6 | |
| A | WO 2017/007903 A1 (FARSIGHT GENOME SYSTEMS INC [US]) 12 January 2017 (2017-01-12) * paragraphs [0118] – [0125] * | 1-6 | |
| A | PEDERSEN BRENT S ET AL: "Mosdepth: quick coverage calculation for genomes and exomes", BIOINFORMATICS, vol. 34, no. 5, 1 March 2018 (2018-03-01), pages 867-868, XP055959545, GB ISSN: 1367-4803, DOI: 10.1093/bioinformatics/btx699 Retrieved from the Internet: URL:https://www.ncbi.nlm.nih.gov/pmc/articles/PMC6030888/pdf/btx699.pdf> * the whole document * | 1-6 | |

TECHNICAL FIELDS SEARCHED (IPC)

G16B

The present search report has been drawn up for all claims

| Place of search | Date of completion of the search | Examiner |
|---|---|---|
| Munich | 11 September 2022 | Thumb, Werner |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 22 16 6649

This annex lists the patent family members relating to the patent documents cited in the above-mentioned European search report.
The members are as contained in the European Patent Office EDP file on
The European Patent Office is in no way liable for these particulars which are merely given for the purpose of information.

11-09-2022

| Patent document cited in search report | | Publication date | Patent family member(s) | | Publication date |
|---|---|---|---|---|---|
| EP 3879537 | A1 | 15-09-2021 | EP | 3879537 A1 | 15-09-2021 |
| | | | WO | 2021180771 A1 | 16-09-2021 |
| WO 2017007903 | A1 | 12-01-2017 | CN | 107922973 A | 17-04-2018 |
| | | | GB | 2555551 A | 02-05-2018 |
| | | | HK | 1252804 A1 | 06-06-2019 |
| | | | US | 2018218789 A1 | 02-08-2018 |
| | | | US | 2020203014 A1 | 25-06-2020 |
| | | | WO | 2017007903 A1 | 12-01-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021180771 A

- WO 2021180768 A

**Non-patent literature cited in the description**

- **AKSAMENTOV, I. et al.** Nextclade: clade assignment, mutation calling and quality control for viral genomes. *Journal of Open Source Software,* 2021, vol. 6 (67), 3773
- **CDC.** *Sars-cov-2 variant classifications and definitions,* 2021, https: //www.cdc.gov/coronavirus/2019-ncov/variants/variantclassifications.html
- **CHARRE, C. et al.** Evaluation of ngs-based approaches for sars-cov-2 whole genome characterisation. *Virus evolution,* 2020, vol. 6 (2), veaa075
- **CHEN, S. et al.** A computational toolset for rapid identification of sars-cov-2, other viruses, and microorganisms from sequencing data. *bioRxiv,* 2020
- **DELAHAYE, C. ; NICOLAS, J.** Sequencing DNA with nanopores: Troubles and biases. *PLoS ONE,* 10 October 2021, vol. 16
- **FRIEDMAN, J. et al.** Regularization paths for generalized linear models via coordinate descent. *Journal of Statistical Software,* 2010, vol. 33 (1), 1-22
- **ITOKAWA, K. et al.** Disentangling primer interactions improves sars-cov-2 genome sequencing by multiplex tiling pcr. *PloS one,* 2020, vol. 15 (9), e0239403
- **JAILLARD, M. et al.** Interpreting k-mer-based signatures for antibiotic resistance prediction. *GigaScience,* 2020, vol. 9 (10), giaa110
- **JOUET, A. et al.** Deep amplicon sequencing for culture-free prediction of susceptibility or resistance to 13 anti-tuberculous drugs. *European Respiratory Journal,* 2021, vol. 57 (3
- **KHARE, S. et al.** Gisaid's role in pandemic response. *China CDC Weekly,* 2021, vol. 3 (49), 1049-1051
- **LIU, T. et al.** A benchmarking study of sars-cov-2 whole-genome sequencing protocols using covid-19 patient samples. *Iscience,* 2021, vol. 24 (8), 102892

- **MAGI, A. et al.** Nanopore sequencing data analysis: state of the art, applications and challenges. *Briefings in Bioinformatics,* June 2017
- **MARÇAIS, G. et al.** Mummer4: A fast and versatile genome alignment system. *PLOS Computational Biology,* 2018, vol. 14 (1), 1-14
- **MEEHAN, C. J. et al.** Whole genome sequencing of mycobacterium tuberculosis: current standards and open issues. *Nature Reviews Microbiology,* 2019, vol. 17 (9), 533-545
- **O'TOOLE et al.** Assignment of epidemiological lineages in an emerging pandemic using the pangolin tool. *Virus Evolution,* 2021, vol. 7 (2), veab064
- **PARUMS, D. V.** Revised world health organization (who) terminology for variants of concern and variants of interest of sarscov- 2. *Medical Science Monitor: International Medical Journal of Experimental and Clinical Research,* 2021, vol. 27, e933622-1
- **QUICK, J. et al.** Multiplex pcr method for minion and illumine sequencing of zika and other virus genomes directly from clinical samples. *Nature protocols,* 2017, vol. 12 (6), 1261-1276
- **TYSON, J. R. et al.** Improvements to the artic multiplex pcr method for sars-cov-2 genome sequencing using nanopore. *BioRxiv,* 2020
- **VAN DORP, L. et al.** Emergence of genomic diversity and recurrent mutations in sars-cov-2. *Infection, Genetics and Evolution,* 2020, vol. 83, 104351
- **WHO.** *Tracking sars-cov-2 variants,* 2021, https://www.who.int/en/activities/tracking-sars-cov-2-variants
- **WU, F. et al.** A new coronavirus associated with human respiratory disease in china. *Nature,* 2020, vol. 579 (7798), 265-269